# EUROPÄISCHE PATENTANMELDUNG

(11) **EP 1 830 192 A1**
(43) Veröffentlichungstag der Anmeldung: **05.09.2007**
(21) Anmeldenummer: 07003837.7
(22) Anmeldetag: 24.02.2007
(51) Int. Cl.: G01N 33/74

(54) **Verfahren zum Aufbewahren des humanen Wachstumshoroms Somatotropin und Verfahren zum quantitativen Nachweis des humanen Wachstumshormons Somatotropin**

(30) Priorität: 28.02.2006 DE 102006009534
(71) Anmelder: Mediagnost Gesellschaft für Forschung und Herstellung von Diagnostika GmbH, 72127 Kusterdingen (DE)
(72) Erfinder: Pridzun, Lutz, Dr., 71083 Herrenberg (DE)
(74) Vertreter: LOUIS, PÖHLAU, LOHRENTZ

(57) **Zusammenfassung**

Die Erfindung betrifft Verfahren zum Aufbewahren von Somatotropin, wobei a) eine isolierte Körperflüssigkeitsprobe, die Somatotropin enthält, auf einen, vorzugsweise flächenförmigen, Träger aufgebracht und gebunden wird.

Die Erfindung betrifft ferner ein Verfahren zum quantitativen Nachweis von Somatotropin, wobei a) eine isolierte Körperflüssigkeitsprobe, die Somatotropin enthält, auf einen, vorzugsweise flächenförmigen, Träger aufgebracht und gebunden wird, b) Freisetzen von Somatotropin aus der in Schritt a) gebundenen Körperflüssigkeitsprobe, c) Inkontaktbringen des in Schritt b) freigesetzten Somatotropins mit einem für Somatotropin spezifischen Nachweisreagenz oder Nachweisreagenzkombination und e) Bestimmen eines Meßwertes, der mit der Somatotropinmenge korreliert.

## Beschreibung

Die vorliegende Erfindung betrifft ein Verfahren zum Aufbewahren von Somatotropin, ein Verfahren zum quantitativen Nachweis von Somatotropin, die Verwendung von einem, vorzugsweise flächenförmigen, Träger zum Binden von Körperflüssigkeit, welche Somatotropin enthält und ein Testsystem zum Nachweis von Somatotropin in einem isolierten Probenmaterial.

Die Bestimmung des humanen Wachstumshormons hWH, welches auch als Somatotropin bezeichnet wird, erfolgt im Rahmen der Diagnostik von Wachstumshormonmangel oder Wachstumshormonexzeß (Akromegalie). Das humane Wachstumshormon ist auch unter der aus dem Englischen abgeleiteten Bezeichnung hGH (human Growth Hormone) bekannt. Während bzw. nach der chirurgischen und/oder medikamentösen Therapie einer Akromegalie werden Somatotropin-Messungen zur Erfolgssicherung eingesetzt. Ferner wird auch der Erfolg einer Wachtumshormontherapie bei kleinwüchsigen Kindern durch die Messung von Somatotropin überwacht.

Das System des Wachstumshormons Somatotropin ist durch eine außerordentliche Komplexität gekennzeichnet. Somatotropin ist das Genprodukt des GH1-Gens auf Chromosom 17 der somatrotopen Hypophysezellen. Das Genprodukt ist ein nicht-glykolysiertes Gen von ca. 22 kDa (191 Aminosäuren). Durch einen alternativen Syntheseweg wird auch eine ca. 20 kDa große GH-Variante gebildet. Etwa 80 % des Wachstumshormons bestehen aus der ca. 22 kDa-Variante, während die restlichen etwa 20 % von der ca. 20 kDa großen Variante gebildet werden. Darüber hinaus gibt es eine Vielfalt kleinerer Varianten, post-translationaler Veränderungen und Aggregate in der Zirkulation. Im Blut liegt Somatotropin (hGH) zudem an ein GH-Bindungsprotein (GHBP) gebunden vor, welches die extrazelluläre Domäne des Membran ständigen Rezeptors ist. Hierdurch kann die Halbwertszeit des Hormons ebenso verändert werden, wie seine zelluläre Interaktion. Das Wachstumshormon Somatotropin ist speziesspezifisch. Die Sekretion des Somatotropins ist ebenso vielfältigen Einflüssen unterworfen.

Spontan erfolgt die Sekretion pulsatil (1 Puls alle 3 Stunden) mit einem Maximums während des Nachtschlafs. Eine Reihe physiologischer, beispielsweise durch eine körperliche Belastung, und artifizieller, beispielsweise durch Hypoglykämie oder Aminosäuren hervorgerufener, Reize führt zur Somatotropin-Sekretion, vermittelt durch die hypothalamischen Hormone Somatostatin und GH-Releasing Hormon (GHRH). Die sezernrierte Menge des Somatotropin wird ferner durch das Alter, Sexualsteroide, den Ernährungszustand, Krankheitszustände und Emotionalität beeinflußt. Wegen dieser vielfältigen Einflußvarianten besteht keine eindeutige Klarheit über das quantitative Sekretionsverhalten. Mehrfachbestimmungen über einen längeren Zeitraum sind unerläßlich.

Die physiologischen Funktionen von Somatotropin sind gleichermaßen vielfältig. Diese Funktionen werden zum Teil durch die vom Somatotropinabhängigen Insulin-ähnlichen Wachstumsfaktoren (IGF), teils unabhängig davon vermittelt. Im Kindes- und Jugendalter ist das Somatotropin-System der Hauptregulator des Wachstums.

Bei einem völligen Fehlen des Somatotropins wird der Mensch nur etwa 1,20 m groß. Darüber hinaus hat Somatotropin einen ausgeprägten anabolen Effekt auf die Muskulatur, Bindegewebe, Knochen und verschiedene spezifische Organe, wie z.B. Herz oder Darm, und wirkt lipolytisch.

Im Kindesalter ist der Mangel an Somatotropin - angeboren oder erworben - die Ursache für einen dauerhaften Kleinwuchs. Beim klinischen Verdacht muß der Somatotropin-Mangel mittels Somatotropin-Stimulationstest oder durch Untersuchung der spontanen Somatotropin -Sekretion nachgewiesen werden. In der Regel werden zunächst IGF1 und IGF-BP-3 bestimmt. Falls diese Werte von Normwerten abweichen, wird eine Differentialdiagnostik mittels Bestimmung des Somatotropins durchgeführt.

Die Ersatztherapie mit rekombinantem menschlichen Somatotropin führt zur Normalisierung des Wachstums. Im Erwachsenenalter ist der Somatotropin - Mangel meist durch Hypophysenadenome als auch deren chirurgische Entfernung bedingt. Der Somatotropin-Mangel des Erwachsenen führt zu einem typischen Krankheitsbild, welches einem vorgezogenem Alterungsprozeß entspricht, wie beispielsweise zu Adipositas, Muskelschwäche, Arteriosklerose, Osteoporose oder Adynamie. Die Ersatzbehandlung ist bei schwerem Somatotropin-Mangel des Erwachsenen eine anerkannte, effektive Therapieform. Die Wirkung der Behandlung wird direkt oder indirekt durch IGF-Messungen und/oder Somatotropin-Messungen im Blut überprüft. Eine exzessive Somatotropin-Sekretion durch ein Hypophysenadenom kann beim Kind zu Gigantismus führen, bei einem Erwachsenen zu einer sogenannten Akromegalie, welches zur Vergrößerung der Akren sowie langfristig zum Diabetes mellitus, einer Herzinsuffizienz und Tumoren führen kann.

Auch Tumore können eine erhöhte Somatotropin-Sekretion bewirken. Die Therapie besteht in der operativen Entfernung des Tumors. Falls diese nicht oder nur unvollständig gelingt, erfolgt eine medikamentöse Therapie mittels Somastatin-Präparaten, welche die Somatotropin-Produktion des Tumors beeinträchtigen, oder neuerdings mittels des Somatotropin-Analogen wie Pegvisumant, welche mit dem Somatotropin-Rezeptor interagieren, jedoch die physiologische Wirkung von Somatotropin nicht hervorrufen.

Außer bei der Erkennung von pathologischen Zuständen wird die Somatotropin-Konzentration auch bei Doping-Kontrollen im Sport überprüft.

Üblicherweise werden Blutproben von Individuen entnommen und bei einer Aufbewahrungstemperatur von 4 °C oder in gefrorenem Zustand in die Untersuchungslabore verschickt. Das Versenden von Proben bei Temperaturen unterhalb der Raumtemperatur, beispielsweise 25 °C, führt häufig zu Fehlern bei der quantitativen Bestimmung, wenn die Kühlkette unterbrochen wird. Ferner ist auch eine Versendung der Proben bei Raumtemperatur aus Kostengründen erstrebenswert.

Aus der DE 37 38 982 A1 ist ein Verfahren zur Bestimmung von Proteinen, Proteinstrukturen und Nukleinsäuren, die aus Proben von Körperflüssigkeiten stammen, bekannt. Bei diesem Verfahren wird die Probe von einem saugfähigen Träger aufgenommen, wobei der saugfähige Träger mit einem die Proteine und Nukleinsäuren stabilisierenden Zusatz versehen ist. Vorzugsweise werden die Körperflüssigkeitsproben ferner mit einem mikrobiziden Mittel versetzt. Nachteilig ist, dass bei dem aus der DE 37 38 982 A1 bekannten Verfahren das Untersuchungsgut mit einem stabilisierenden Zusatz versehen und die Körperflüssigkeitsprobe in der Regel mit einem mikrobiziden Mittel versehen werden muss.

Aus der EP 1 136 068 A2 ist ein Verfahren zur Stabilisierung eines physiologisch aktiven Peptides bekannt. Bei diesem Verfahren wird eine das physiologisch aktive Peptid enthaltende flüssige Probe mit einem Stabilisierungsmittel versetzt und nachfolgend unter Erhalt eines Pulvers getrocknet. Auch dieses Verfahren ist nachteiligerweise aufwendig, da das Probenmaterial zunächst mit einem Stabilisierungsmittel versetzt und nachfolgend unter Erhalt des gewünschten Pulvers getrocknet werden muss.

Aufgabe der Erfindung ist es, ein Verfahren zum Aufbewahren des humanen Wachstumshormons Somatotropin bereitzustellen, das eine Aufbewahrung des Somatotropins aus einer Körperflüssigkeitsprobe auch bei Raumtemperatur auf einfache Art und Weise ermöglicht, ohne daß sich die Somatotropin-Konzentration in der Probe verändert.

Eine weitere Aufgabe der Erfindung ist es, ein Verfahren zum Aufbewahren des humanen Wachstumshormons Somatotropin bereitzustellen, das die Aufbewahrung und/oder den Transport der Somatotropin enthaltenden Proben in stabiler Form ermöglicht

Eine weitere Aufgabe der Erfindung ist es, ein Verfahren zum quantitativen Nachweis des humanen Wachstumshormons Somatotropin bereitzustellen, welches die quantitative Bestimmung des humanen Wachstumshormons Somatotropin auch aus bei Raumtemperatur gelagerten Körperflüssigkeitsproben gewährleistet.

Die Verfahren sollen dabei einfach und zuverlässig durchzuführen sein.

Die der Erfindung zugrundeliegende Aufgabe wird durch ein Verfahren zum Aufbewahren des humanen Wachstumshormons Somatotropin gelöst, wobei
a) eine isolierte Körperflüssigkeitsprobe, die Somatotropin enthält, auf einen, vorzugsweise flächenförmigen, Träger aufgebracht und gebunden wird.
   Die der Erfindung zugrundeliegende Aufgabe wird auch durch ein Verfahren zum quantitativen Nachweis von Somatotropin gelöst, das die folgenden Schritte umfasst:
b) Freisetzen von Somatotropin von einer auf einem, vorzugsweise flächenförmigen, Träger gebundenen Körperflüssigkeitsprobe,
c) Inkontaktbringen des in Schritt b) freigesetzten Somatotropins mit einem für Somatotropin spezifischen Nachweisreagenz oder Nachweisreagenzkombination,
d) Bestimmen eines Messwertes, der mit der Somatotropinmenge korreliert.

Die der Erfindung zugrundeliegende Aufgabe wird ferner durch ein Verfahren zum quantitativen Nachweis des humanen Wachstumshormons Somatotropin gelöst, wobei
a) eine isolierte Körperflüssigkeitsprobe, die Somatotropin enthält, auf einen, vorzugsweise flächenförmigen, Träger aufgebracht und gebunden wird,
b) Somatotropin aus der in Schritt a) gebundenen Körperflüssigkeitsprobe freigesetzt wird,
c) das in Schritt b) freigesetzte Somatotropin mit einem für Somatotropin spezifischen Nachweisreagenz oder einer spezifischen Nachweisreagenzkombination in Kontakt gebracht wird, und
d) Bestimmen eines Meßwertes, der mit der Somatotropin-Menge korreliert.

Bei der vorliegenden Erfindung hat sich völlig überraschend gezeigt, dass Somatotropin in einer an einen Träger gebundenen Form außerordentlich stabil ist. Somit ist es bei der vorliegenden Erfindung nicht notwendig, der isolierten Körperflüssigkeitsprobe oder dem Träger Stabilisierungsmittel hinzuzufügen. Das in der isolierten Körperflüssigkeitsprobe enthaltene Somatotropin wird mithin auf einen Träger aufgebracht und von diesem gebunden, ohne dass zugleich Stabilisierungsmittel für Somatotropin in der Körperflüssigkeitsprobe oder in dem bzw. auf dem Träger vorhanden sein müssen. Die Somatotropinhaltige Körperflüssigkeitsprobe liegt vorzugsweise in einer getrockneten Form ohne Stabilisierungsmittel für Somatotropin auf dem, vorzugsweise flächenförmigen, Träger vor. Das Somatotropin ist in der getrockneten und an einen, vorzugsweise flächenförmigen, Träger gebundenen Form außerordentlich stabil und kann somit über einen sehr großen Zeitraum von mehreren Monaten bis Jahren aufbewahrt werden.

Als isolierte Körperflüssigkeitsprobe wird vorzugsweise Vollblut, Blutplasma, Serum, Cerebrospinalflüssigkeit, Sputum, Urin, Muttermilch, Sperma oder Gemische davon verwendet. Es ist selbstverständlich auch möglich, die jeweilige Körperflüssigkeitsprobe vor dem Inkontaktbringen mit dem, vorzugsweise flächigen, Träger im Hinblick auf Somatotropin teilweise oder vollständig zu reinigen. Beispielsweise kann das Somatotropin vor dem Aufbringen bzw. Binden an den, vorzugsweise flächenförmigen, Träger in einen Puffer überführt werden, wenn dies bei dem Messverfahren zur Bestimmung der Somatotropinmenge von Vorteil ist, wenn sich hierdurch beispielsweise eine Erhöhung der Messgenauigkeit erwirken lässt. Vorzugsweise wird die isolierte Körperflüssigkeitsprobe jedoch direkt auf den, vorzugsweise flächenförmigen, Träger aufgebracht und gebunden.

Unter dem Begriff Somatotropin im Sinne der Erfindung wird zum einen das nicht-glykolysierte ca. 22 kDa Protein des Somatotropin-Genprodukts sowie zum anderen die durch den alternativen Syntheseweg gebildete ca. 20 kDa Somatotropin-Variante, biologisch aktive Derivate derselben, post-translational veränderte Proteine derselben und Aggregate in der Blutzirkulation, wie beispielsweise an das Bindungsprotein gebundenes Somatotropin verstanden.

Die Proteine können aber auch in trunkierter Form vorliegen, beispielsweise nach einer proteolytischen Degradation.

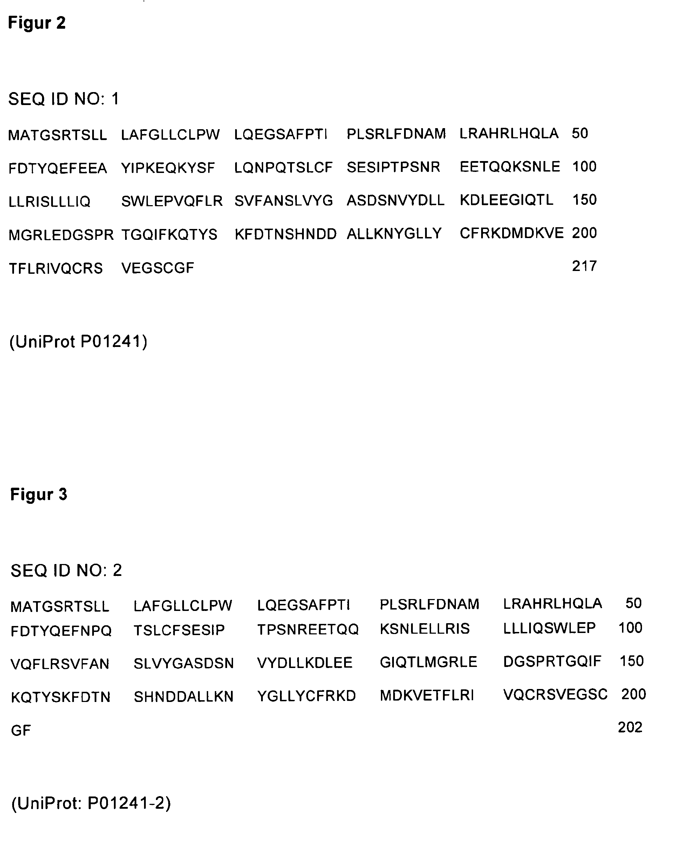

In Figur 2 ist SEQ ID NO. 1 wiedergegeben, die die Sequenz des Vorläufers der etwa 22 kDa-Variante von Somatotropin (precursor) darstellt. Im reifen Protein ist die Signalsequenz, die die ersten Aminosäuren 1 bis 26 umfaßt, abgespalten. Die 22 kDa-Variante des Somatotropins umfaßt mithin die Aminosäuren 27 bis 217.

In Figur 3 ist SEQ-ID NO. 2 wiedergegeben, die die Sequenz des Vorläufers der etwa 20 kDa-Variante von Somatotropin (precursor) darstellt. Im reifen Protein ist die Signalsequenz, die die ersten Aminosäuren 1 bis 26 umfaßt, abgespalten. Die 22 kDa-Variante des Somatotropins umfaßt mithin die Aminosäuren 27 bis 202.

Bei der 20 kDa-Variante fehlen in bezug auf die 22 kDa-Variante die Aminosäuren 58 bis 72 der SEQ ID NO. 2. Das Somatotropin mit der SEQ ID NO. 2 stellt eine zweite Isoform des Somatotropins mit der SEQ ID NO. 1 dar.

Von Somatotropin sind wenigstens zwei weitere Isoformen bekannt, wobei bei der dritten Isoform die Aminosäuren 111 bis 148 und bei der vierten Isoform die Aminosäuren 117 sowie 162 fehlen. Die Angaben der Aminosäurenpositionen beziehen sich dabei jeweils auf SEQ ID NO. 1.

Unter Somatotropin werden im Sinne der vorliegenden Erfindung sämtliche Isoformen von Somatotropin verstanden, die gegebenenfalls postranslational modifiziert, beispielsweise trunkiert sein können.

Die verschiedenen Aminosäurepositionen in den verschiedenen Somatotropinvarianten sind nachfolgend in Tabelle 1 zusammenfassend dargestellt, wobei sich sämtliche Angaben auf SEQ ID NO. 1 beziehen.

**Tabelle 1:**

| | Aminosäuren, bezogen auf SEQ ID NO. 1 | Deletierte Aminosäuren, bezogen auf SEQ ID NO. 1 |
|---|---|---|
| Signalsequenz | 1-26 | |
| Somatotropin | 27-217 | |
| Somatotropin Isoform 2 | 27 bis 57 und 73 bis 217 | 58-72 fehlen |
| Somatotropin Isoform 3 | 27 bis 110 und 149 bis 217 | 111-148 fehlen |
| Somatotropin Isoform 4 | 27 bis 116 und 118 bis 161 und 163 bis 217 | 117 und 162 fehlen |

Es hat sich überraschenderweise gezeigt, daß das sehr instabile Protein des Somatotropins nach dem Aufbringen auf einen, vorzugsweise flächenförmigen, Träger auch bei Raumtemperatur über einen längeren Zeitraum stabil ist und nachfolgend quantitativ vermessen werden kann. Insbesondere hat sich gezeigt, daß das in einer isolierten Körperflüssigkeitsprobe enthaltene Somatotropin auf einen, vorzugsweise flächenförmigen, Träger aufgebracht und von diesem gebunden werden kann. Es hat sich überraschend gezeigt, daß das gebundene Somatotropin von dem, vorzugsweise flächenförmigen, Träger ohne wesentliche quantitative Verluste freigesetzt und sodann bestimmt werden kann. Ferner hat sich gezeigt, daß eine isolierte Körperflüssigkeitsprobe, die Somatotropin enthält, auf einen, vorzugsweise flächenförmigen, Träger aufgebracht und gebunden werden kann und bei Raumtemperatur über einen längeren Zeitraum von bis zu fünf Jahren gelagert werden kann und anschließend das Somatotropin von dem, vorzugsweise flächenförmigen Träger, quantitativ freigesetzt und sodann bestimmt werden kann.

Vorteilhaft erlaubt somit das erfindungsgemäße Verfahren eine vereinfachte Lagerung bzw. Aufbewahrung und Versendung von Körperflüssigkeitsproben, aus denen der Somatotropingehalt bestimmt werden soll. Bspw. können die Körperflüssigkeitsproben auf einen flächenförmigen Träger, vorzugsweise in Postkartenformat, aufgebracht und, vorzugsweise nach Trocknung, per Post ohne weitere Kühlung verschickt werden. Ferner ist die Aufbewahrung von Körperflüssigkeitsproben, die auf einem, vorzugsweise flächenförmigen, Träger gebunden sind, auch aus Sicherheitsgründen von Vorteil, da keine infektiösen Körperflüssigkeiten beim Verschicken der Proben auslaufen können.

Nach der Aufbringung der Körperflüssigkeitsproben auf den, vorzugsweise flächenförmigen Träger, erfolgt vorzugsweise eine Trocknung. Es ist selbstverständlich auch möglich, den, vorzugsweise flächenförmigen, Träger in feuchter bzw. teilweise getrockneter Form zu versenden.

Es ist bevorzugt, einen flächenförmigen Träger zu verwenden. Selbstverständlich können auch andere Trägerformen, beispielsweise in Pulverfrom, Gelform, in komprimierter oder gepreßter Form, beispielsweise in Form einer Tablette oder einer Granulats, verwendet werden. Diese weiteren Träger weisen dabei vorzugsweise ein Adsorptions- und/oder Absorptionsvermögen für Somatotropin auf.

Bevorzugte Weiterbildungen der erfindungsgemäßen Verfahren nach Anspruch 1 oder 2 oder 3 sind in den Ansprüchen 4 bis 17 angegeben.

Das erfindungsgemäße Verfahren wird nicht am Menschen oder Tier durchgeführt. Das erfindungsgemäße Verfahren wird ausschließlich *in vitro* durchgeführt.

Bei einer Ausführungsform wird die Körperflüssigkeit durch Absorption und/oder Adsorption gebunden.

Unter dem Begriff isolierte Körperflüssigkeitsprobe im Sinne der Erfindung wird eine Körperflüssigkeitsprobe verstanden, die einem Menschen entnommen wurde.

Bei einer Ausführungsform werden Körperflüssigkeitsproben verwendet, die aus der Gruppe ausgewählt sind, die aus Vollblut, Blutplasma, Serum, Cerebrospinalflüssigkeit, Sputum, Urin, Muttermilch, Sperma, Biopsie-Eluat und Gemischen davon besteht. Vorzugsweise wird Vollblut, Blutplasma und/oder Serum als Körperflüssigkeit verwendet.

Bei einer Ausführungsform bindet der vorzugsweise verwendete flächenförmige Träger pro Flächeneinheit eine definierte Menge Körperflüssigkeit. Vorzugsweise bindet der flächenförmige Träger vorzugsweise 0,5 µl bis 100 µl, bevorzugt 0,7 µl bis 50 µl, noch weiter bevorzugt 0,8 µl bis 1 µl, beispielsweise 1 bis 2 µl Körperflüssigkeit auf einer kreisrunden Scheibe mit einem Durchmesser von vorzugsweise 1 mm bis 20 mm, bevorzugt von 1,5 mm bis 10 mm, weiter bevorzugt von 2 bis 5 mm, beispielsweise von 3,18 mm Durchmesser.

Bei einer Ausführungsform wird das Somatotropin aus der in Schritt a) gebundenen Körperflüssigkeitsprobe durch eine Elutionsflüssigkeit freigesetzt.

Gemäß einer bevorzugten Weiterbildung der Erfindung werden Schritt b) und Schritt c) bei dem Verfahren zum quantitativen Nachweis von Somatotropin in einem Schritt zusammengefaßt. Mit der Freisetzung des in Schritt a) gebundenen Somatotropins erfolgt unmittelbar das Inkontaktbringen mit dem Nachweisreagenz. Der Nachweis kann beispielsweise über Lumineszenz, vorzugsweise Fluoreszenz oder Chemilumineszenz, oder Radioaktivität erfolgen.

Bei der Elutionsflüssigkeit handelt es sich vorzugsweise um einen Puffer, beispielsweise einen Phosphatpuffer oder Tris-Puffer, der ferner einen oder mehrere Konservierungsstoffe, wie Natriumazid, sowie Natriumchlorid und/oder BSA (Rinderserumalbumin) enthalten kann, und wenigstens ein Tensid, vorzugsweise wenigstens ein nicht-ionogenes Tensid, wie beispielsweise Triton X-100, Triton X-200, Tween-20, Tween 80, NP-40, etc. oder auch Mischungen davon umfasst. Der Puffer weist vorzugsweise einen pH-Wert im Bereich von 6 bis 8, weiter bevorzugt von 7 bis 8 auf. Gemäß einer Variante der Erfindung besteht die Elutionsflüssigkeit aus 45 bis 55 mM Natriumphosphat, 90 bis 110 mM Natriumchlorid, 0,04 bis 0,06 (w/v) Natriumazid, 0,15 bis 0,25 (w/v) BSA, 0,08 bis 1,2 (v/v) Triton X-100, pH 7,2 bis 7,6).

Zum Freisetzen des Somatotropins von dem Träger werden vorzugsweise 5 bis 1000 µl, weiter bevorzugt von 50 bis 500 µl, noch weiter bevorzugt von 100 bis 400 µl, beispielsweise 200 bis 300 µl, Elutionsflüssigkeit pro Flächeneinheit, vorzugsweise von 1 bis 300 mm², bevorzugt 3 bis 100 mm², noch weiter bevorzugt von 5 bis 20 mm², beispielsweise 8 mm² Träger verwendet.

Bei einer bevorzugten Ausführungsform ist der flächenförmigen Träger ein Filterpapier. Der flächenförmige Träger kann aus Nitrocellulose, PVDF oder Baumwollfasern, insbesondere Linters, hergestellt sein.

Vorzugsweise besteht der flächenförmige Träger zu mindestens 90 Gew.-% bezogen auf die Gesamtmenge des flächenförmigen Trägers aus Linters, am meisten bevorzugt besteht der flächenförmige Träger zu 100 Gew.-% bezogen auf die Gesamtmenge des flächenförmigen Trägers aus Linters ohne weitere Additive. Vorzugsweise weisen die Linterfasern eine Länge von 2 bis 3 mm auf. Beispielsweise kann als flächenförmiger Träger das Filterpapier No. 903^{®} von Schleicher & Schuell, Deutschland verwendet werden.

Linters sind unspinnbare kurze Baumwollfasern, die nach dem Entfernen der Leinentextilfasern auf dem Samen bleiben und in der Ölmühle vor dem Auspressen des Baumwollsamenöls in einen oder mehreren Schnitten abgetrennt werden. Während die längeren Fasern des ersten Schnitts (3 bis 6 mm) für Polster und Matratzen verwendet werden, werden die kürzeren (2 bis 3 mm) Fasern des zweiten Schnitts für die Herstellung für reinste Cellulose (Zellstoff) insbesondere für Filter verwendet, die für den Zweck der vorliegenden Erfindung hervorragend geeignet sind.

Vorzugsweise wird nach Schritt a) und ggf. vor Schritt b) des erfindungsgemäßen Verfahrens aus dem flächenförmigen Träger eine definierte Teilfläche entfernt, auf welche die Körperflüssigkeitsprobe aufgebracht wurde. Bspw. wird die definierte Fläche ausgeschnitten oder ausgestanztVorzugsweise wird eine kreisrunde Fläche mit einem Durchmesser von 2 bis 4 mm für die Messung entfernt.

Vorzugsweise wird nach Schritt a) oder vor Schritt b) der flächenförmige absorptions- und/oder adsorptionsfähige Träger und/oder die entfernte definierte Fläche des absorptions- und/oder adsorptionsfähigen Trägers getrocknet. Die Trocknung erfolgt vorzugsweise bei Raumtemperatur, beispielsweise bei einer Temperatur von 15 bis 30 °C.

Bei einer bevorzugten Ausführungsform ist das spezifische Nachweisreagens ein Antikörper oder Rezeptor für Somatotropin. Für die Zwecke der vorliegenden Erfindung haben sich polyklonale Antikörper gegen Somatotropin, beispielsweise aus Kaninchen gegen die ca. 22 kDa Variante gewonnene polyklonale Antikörper, als sehr geeignet erwiesen. Verwendbar ist auch der von GenWay Biotech, Inc., USA, vertriebene aus Hühnchen gewonnene polyklonale IgY-Antikörper anti-hGH (Katalognummer A10062F).

Es können aber auch monoklonale Antikörper gegen Somatotropin, beispielsweise ab9821, ab15317, ab9822, ab15318, ab8490, ab765, ab1953, ab1956 und/oder ab1954 der Firma Abcam plc, Cambridge, England verwendet werden.

Vorzugsweise wird der Meßwert durch ELISA, RIA, LIA und/oder IFA nach Elution von dem, vorzugsweise flächenförmigen, Träger bestimmt.

Unter IFA (Immunfunktionaler Assay) wird ein immunologischer Assay verstanden, der vorzugsweise auf zwei spezifischen, vorzugsweise monoklonalen, Antikörpern basiert. Die beiden Antikörper erkennen die Rezeptorbindungsstellen im Somatotropin, die für die Signalkaskade notwendig sind.

Unter LIA (Lumineszenzimmunoassay) wird ein immunologischer Assay verstanden, bei dem das gemessene Signal auf einer Chemilumineszenzreaktion beruht.

Bei einer Ausführungsform kann das Somatotropin wie folgt durch einen Sandwich-Assay bestimmt werden:

Hochaffine spezifische Antikörper, beispielsweise polyklonale Kaninchenantikörper, werden bei einem Sandwich-Assay an eine Mikrotiterplatte gekoppelt, welche das Somatotropin aus dem Eluat binden. In einem nachfolgenden Schritt wird das gebundene Somatotropin von einem Zweitantikörper, beispielsweise einem biotinylierten Anti-Somatotropin-Antiserum erkannt. Danach bindet ein Streptavidin-Nachweisreagenzkonjugat, beispielsweise ein Streptravidin-Peroxidase-Enzymkonjugat, hochspezifisch das Biotin des Anti-Somatotropin-Antiserums und katalysiert in der abschließenden Substratreaktion den Farbumschlag, quantitativ abhängig vom Somatotropin-Gehalt der Proben. Die verwendeten Standards können dabei aus rekombinantem humanen Wachstumshormon in Konzentrationen von 50, 150, 300, 600 bis 1000 pg/ml bestehen. Als Standardmaterial kann der zweite internationale Standard für Somatotropin NIBSC Code 98/574 verwendet werden. Dieser wurde in einer internationalen Studie im Jahre 2001 mit drei internationalen Einheiten pro mg Protein (3 IU/mg) definiert.

Beispielsweise kann das Somatotropin mit dem kommerziell erhältlichen hGH-Sensitiv ELISA der Firma-Mediagnost bestimmt werden.

Bei einer bevorzugten Ausführungsform der erfindungsgemäßen Verfahren wird der, vorzugsweise flächenförmige, Träger nach Schritt a) und ggf. vor Schritt b) bei Raumtemperatur, vorzugsweise bei einer Temperatur von 10 bis 30°C, gelagert. Vorzugsweise wird der, bevorzugt flächenförmige, Träger nach Schritt a) und ggf. vor Schritt b) für einen Zeitraum von 1 Sekunde bis zu 5Jahren, weiter bevorzugt von 15 Minuten bis zu einem Jahr, noch weiter bevorzugt von 1 Stunde bis zu drei Monaten, beispielsweise von 1 Woche bis zu einem Monat gelagert. Besonders bevorzugt wird der flächenförmige Träger für einen Zeitraum von zwei bis zu drei Wochen gelagert.

Die der Erfindung zugrundeliegende Aufgabe wird auch durch die Verwendung eines flächenförmigen Trägers zum Binden von Körperflüssigkeit, welche Somatotropin umfaßt, gelöst. Vorzugsweise wird der flächenförmige Träger ferner zur Aufbewahrung oder Konservierung des Somatotropins in der Körperflüssigkeit verwendet.

Die der Erfindung zugrundeliegende Aufgabe wird auch durch ein Testsystem mit den Merkmalen des Anspruchs 20 gelöst.

Die in bezug auf das Verfahren vorstehend gemachten Ausführungen gelten entsprechend für das erfindungsgemäße Testsystem und für die erfindungsgemäße Verwendung.

Durch das erfindungsgemäße Verfahren zum Aufbewahren des humanen Wachstumshormons Somatotropin bzw. zum quanitativen Nachweis des humanen Wachstumshormons sowie durch das erfindungsgemäße Testsystem wird die Bestimmung von Somatotropin aus isolierten Körperflüssigkeitsproben wesentlich vereinfacht und verbessert. Die isolierten Körperflüssigkeitenwerden dabei direkt nach erfolgter Entnahme aus dem Patienten auf den, vorzugsweise flächenförmigen, Träger aufgebracht und bevorzugt unter Trocknung gebunden. Überraschenderweise unterliegt das Somatotropin in dieser gebundenen und bevorzugt getrockneten Form keiner Zersetzung und erlaubt völlig überraschend eine zuverlässige quantitative Bestimmung des Somatotropingehaltes.

### Figuren

**Figur 1** zeigt die Korrelation zwischen dem Somatotropin-Gehalt der aus Filterpapierproben und Serumproben bestimmt wurde. Die X-Achse zeigt die Konzentration von Somatotropin in Serumproben [ng/ml]. Die Y-Achse zeigt die Konzentration von Somatotropin aus Filterpapiereluat [ng/µl].
**Figur 2** zeigt SEQ ID NO. 1, die Sequenz der etwa 22 kDa-Variante von Somatotropin.
**Figur 3** zeigt SEQ ID NO. 2, die Sequenz der etwa 20 kDa-Variante von Somatotropin.

### Beispiele:

### Beispiel 1

### Filterpapierproben

Zur Herstellung der Filterproben wird direkt nach der Blutentnahme das Blut aus etwa 1 bis 2 cm Höhe in die Mitte einer mit einem Kreis mit einem Kreisdurchmesser von 1,5 cm markierten Filterkarte aus Filterpapier No. 903^{®} von Schleicher & Schuell, Deutschland, aufgetropft, bis sich der Blutfleck etwa 1,5 cm im Durchmesser ausgebreitet hat. Das Filterpapier wird für 2 bis 4 Stunden bei Raumtemperatur luftgetrocknet. Aus den getrockneten Blutproben wird anschließend der Gehalt an Somatotropin bestimmt. Hierfür werden Filterscheiben mit einem Durchmesser von 3,18 mm aus dem Blutfleck ausgestanzt. Die Filter werden mit 250 µl Puffer (pH 7,4, 50 mM Natriumphosphat, 100 mM Natriumchlorid, 0,095 % (w/v) Germall II^{®}, 0,095 % (w/v) Kathon CG^{®}, 0,5 % (w/v) BSA, 0,05 % (w/v) Tween-20) für 1,5 hrs unter Schütteln extrahiert.

### Beispiel 2

### Serumproben oder Plasmaproben

Das Serum oder das Plasma wird dabei aus dem Vollblut unter Verwendung von handelsüblichen Serumröhrchen oder EDTA-Röhrchen, Citrat-Röhrchen oder Heparin-Röhrchen gemäß Herstellerangaben gewonnen.

### Beispiel 3

### Somatotropin ELISA

Die Proben wurden mit den hGH Sensitiv ELISA der Firma Mediagnost (Reutlingen/Germany) gemäß Herstellerangaben gemessen.

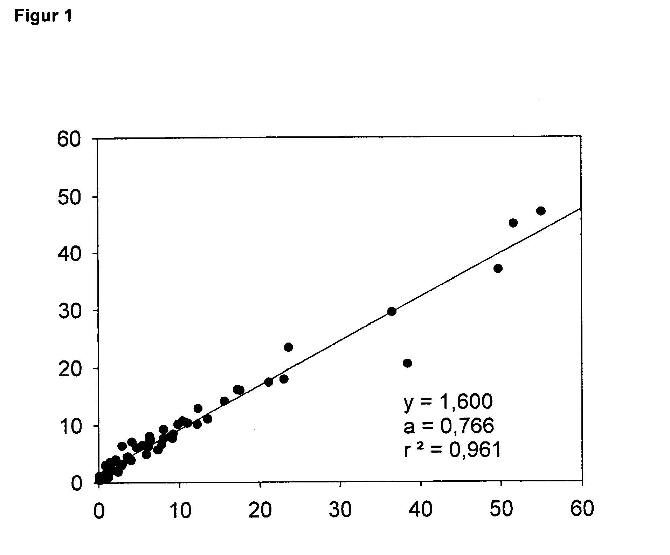

Figur 1 zeigt die Korrelation der Somatotropin-Meßwerte aus 41 Patientenproben, wobei die Somatotropin-Werte aus einer Probe parallel aus Serum bzw. aus Filterpapiereluaten bestimmt wurden. Die bestimmte Konzentration des Somatotropins aus den Filterpapiereluaten ist um einen konstanten Faktor von etwa 5 bis 10 % niedriger als die aus den Serumproben direkt bestimmte Konzentration, korreliert aber direkt in einem linearen Verhältnis mit der Konzentration des Wachstumshormons in den Serumproben. Somit ist die Messung des Wachstumshormons Somatotropin aus getrockneten Filterpapierproben hervorragend geeignet, um das Wachstumshormon in Patientenproben zu bestimmen. Die aus den Filterpapiereluaten bestimmten Meßwerte können mittels eines Korrekturfaktors an die aus den Serumproben erhaltenen Werte angeglichen werden, so daß die aus den Filterpapierproben erhaltenen Meßwerte auch mit anderen Serumproben direkt vergleichbar sind.

## Patentansprüche

1. Verfahren zum Aufbewahren von Somatotropin, wobei
a) eine isolierte Körperflüssigkeitsprobe, die Somatotropin enthält, auf einen, vorzugsweise flächenförmigen, Träger aufgebracht und gebunden wird.

2. Verfahren zum quantitativen Nachweis von Somatotropin, das die folgenden Schritte umfasst:
b) Freisetzen von Somatotropin von einer auf einem, vorzugsweise flächenförmigen, Träger gebundenen Körperflüssigkeitsprobe,
c) Inkontaktbringen des in Schritt b) freigesetzten Somatotropins mit einem für Somatotropin spezifischen Nachweisreagenz oder Nachweisreagenzkombination,
d) Bestimmen eines Messwertes, der mit der Somatotropinmenge korreliert.

3. Verfahren zum quantitativen Nachweis von Somatotropin, wobei
a) eine isolierte Körperflüssigkeitsprobe, die Somatotropin enthält, auf einen, vorzugsweise flächenförmigen, Träger aufgebracht und gebunden wird,
b) Freisetzen von Somatotropin aus der in Schritt a) gebundenen Körperflüssigkeitsprobe,
c) Inkontaktbringen des in Schritt b) freigesetzten Somatotropins mit einem für Somatotropin spezifischen Nachweisreagenz oder Nachweisreagenzkombination,
d) Bestimmen eines Meßwertes, der mit der Somatotropinmenge korreliert.

4. Verfahren nach einem derAnsprüche 1 bis 3, wobei die Körperflüssigkeit durch Absorption und/oder Adsorption von dem, vorzugsweise flächenförmigen, Träger gebunden wird.

5. Verfahren nach einem der vorhergehenden Ansprüche, wobei die isolierte Körperflüssigkeitsprobe Vollblut, Blutplasma, Serum, Cerebrospinalflüssigkeit, Sputum, Urin, Muttermilch, Sperma oder Gemische davon sind.

6. Verfahren nach einem der vorherigen Ansprüche, wobei der, vorzugsweise flächenförmige, Träger pro Flächeneinheit eine definierte Menge Körperflüssigkeit bindet.

7. Verfahren nach einem der Ansprüche 2 bis 6, wobei das Somatotropin aus der in Schritt a) gebundenen Körperflüssigkeitsprobe durch eine Elutionsflüssigkeit freigesetzt wird.

8. Verfahren nach einem der vorhergehenden Ansprüche, wobei der, vorzugsweise flächenförmige, Träger ein Filterpapier ist.

9. Verfahren nach einem der vorhergehenden Ansprüche, wobei der, vorzugsweise flächenförmige, Träger zu mindestens 90 Gew.-% bezogen auf das Gesamtgewicht des, vorzugsweise flächenförmigen, Trägers Linters umfasst.

10. Verfahren nach einem der vorhergehenden Ansprüche, wobei nach Schritt a) und gegebenenfalls vor Schritt b) aus dem, vorzugsweise flächenförmigen, Träger eine definierte Teilfläche entfernt wird, auf welche die Körperflüssigkeitsprobe aufgebracht ist.

11. Verfahren nach einem der vorhergehenden Ansprüche, wobei nach Schritt a) und gegebenenfalls vor Schritt b) der, vorzugsweise flächenförmige, adsorptions- und/oder absorptionsfähige Träger und/oder die entfernte Teilfläche des adsorptions- und/oder absorptionsfähigen Träger getrocknet wird.

12. Verfahren nach Anspruch 11, wobei das spezifische Nachweisreagenz ein Antikörper und oder Rezeptor für Somatotropin ist.

13. Verfahren nach einem der Ansprüche 2 bis 12, wobei der Meßwert durch ELISA, RIA, LIA und/oder IFA bestimmt wird.

14. Verfahren nach einem der vorhergehenden Ansprüche, wobei der, vorzugsweise flächenförmige, Träger nach Schritt a) oder vor Schritt b) bei Raumtemperatur, vorzugsweise bei einer Temperatur von 10 bis 30 °C, aufbewahrt wird.

15. Verfahren nach einem der vorhergehenden Ansprüche, wobei der, vorzugsweise flächenförmige, Träger nach Schritt a) oder vor Schritt b) für einen Zeitraum von 1 Sekunde bis zu fünf Jahren aufbewahrt wird.

16. Verfahren nach Anspruch 15, wobei der, vorzugsweise flächenförmige, Träger für einen Zeitraum von 1 Stunde bis zu einem 1 Jahr gelagert wird.

17. Verfahren nach einem der Ansprüche 2 bis 16, wobei Schritt b) und schritt c) in einem Schritt durchgeführt werden.

18. Verwendung von einem, vorzugsweise flächenförmigen, Träger zum aufbewahren und Binden von Körperflüssigkeit, welche Somatotropin umfasst.

19. Verwendung nach Anspruch 18, wobei der, vorzugsweise flächenförmige, Träger ferner zur Konservierung des Somatotropins in der Körperflüssigkeit verwendet wird.

20. Testsystem zum Nachweis von Somatotropin in einem isolierten Probenmaterial, umfassend
a) einen, vorzugsweise flächenförmigen, Träger, welcher zum Aufbewahren und Binden von Somatotropin aus einer isolierten Körperflüssigkeitsprobe geeignet ist,
b) eine Elutionsflüssigkeit, um das auf dem flächenförmigen Träger gebundene Somatotropin freizusetzen,
c) ein für Somatotropin spezifisches Nachweisreagenz oder eine spezifische Nachweisreagenzkombination.


SEQUENCE LISTING

<110> Mediagnost Gesellschaft für Forschung und Herstellung von
      Diagnostika GmbH

<120> Verfahren zum Aufbewahren des humanen Wachstumsfaktors
      Somatotropin und Verfahren zum quantitativen Nachweis des
      humanen Wachstumsfaktors Somatotropin

<130> 47818ep

<140>
<141>

<160> 2

<170> PatentIn version 3.3

<210> 1
<211> 217
<212> PRT
<213> human

<400> 1

<210>  2
<211>  202
<212>  PRT
<213>  human

<400>  2

Met Ala Thr Gly Ser Arg Thr Ser Leu Leu Leu Ala Phe Gly Leu Leu
1               5                   10                  15

Cys Leu Pro Trp Leu Gln Glu Gly Ser Ala Phe Pro Thr Ile Pro Leu
            20                  25                  30

Ser Arg Leu Phe Asp Asn Ala Met Leu Arg Ala His Arg Leu His Gln
        35                  40                  45

Leu Ala Phe Asp Thr Tyr Gln Glu Phe Asn Pro Gln Thr Ser Leu Cys
    50                  55                  60

Phe Ser Glu Ser Ile Pro Thr Pro Ser Asn Arg Glu Glu Thr Gln Gln
65                  70                  75                  80

Lys Ser Asn Leu Glu Leu Leu Arg Ile Ser Leu Leu Leu Ile Gln Ser
                85                  90                  95

Trp Leu Glu Pro Val Gln Phe Leu Arg Ser Val Phe Ala Asn Ser Leu
            100                 105                 110

Val Tyr Gly Ala Ser Asp Ser Asn Val Tyr Asp Leu Leu Lys Asp Leu
        115                 120                 125

Glu Glu Gly Ile Gln Thr Leu Met Gly Arg Leu Glu Asp Gly Ser Pro
    130                 135                 140

Arg Thr Gly Gln Ile Phe Lys Gln Thr Tyr Ser Lys Phe Asp Thr Asn
145                 150                 155                 160

Ser His Asn Asp Asp Ala Leu Leu Lys Asn Tyr Gly Leu Leu Tyr Cys
                165                 170                 175

Phe Arg Lys Asp Met Asp Lys Val Glu Thr Phe Leu Arg Ile Val Gln
            180                 185                 190

Cys Arg Ser Val Glu Gly Ser Cys Gly Phe
        195                 200